# EUROPEAN PATENT SPECIFICATION

(11) **EP 2 303 129 B1**
(45) Date of publication and mention of the grant of the patent: **14.08.2013**
(21) Application number: 09762879.6
(22) Date of filing: 09.06.2009
(51) Int. Cl.: A61B 7/02, A61B 19/02, A61B 19/08

(54) **DISPOSABLE STETHOSCOPE GLOVE, INSTALLATIONKIT AND METHOD FOR INSTALLATION**
EINWEG-STETHOSKOP-HANDSCHUH, INSTALLATIONSKIT UND VERFAHREN ZUR INSTALLATION
PROTECTION JETABLE POUR STÉTHOSCOPE, KIT D'INSTALLATION ET PROCÉDÉ ASSOCIÉ

(30) Priority: 10.06.2008 US 60124 P
(43) Date of publication of application: 06.04.2011
(73) Proprietor: Manlapaz, Eller, Gig Harbor, WA 98332 (US)
(72) Inventor: Manlapaz, Eller, Gig Harbor, WA 98332 (US)
(74) Representative: Leeming, John Gerard
(86) International application number: PCT/US2009/003474
(87) International publication number: WO 2009/151583

(56) References cited:
- US-A- 3 738 479
- US-A- 5 365 023
- US-A- 5 428 193
- US-A- 5 466 897
- US-A1- 2007 045 039
- US-B1- 6 520 281

## Description

### Field of the Invention

This invention pertains generally to medical accessories and more particularly to a disposable glove for stethoscope bells and diaphragms for use by all medical providers and also an apparatus and method for installing the glove on a stethoscope.

### Background of the Field

As a universal precaution to prevent disease transmission, hand sanitizing has been actively promoted in all medical institutions. This practice prevents the human hands from becoming vehicles of diseases or bodily fluid transmission. There are three ways to accomplish this: (1) by hand washing with soap and running water, (2) by applying antimicrobial gel, and (3) by shielding hands with a disposable, single-use device such as a glove.

In this day and age, dissemination of diseases, particularly infection, has been a paramount concern. Practicing universal precaution by everyone in the medical community can help eliminate serious healthcare-associated and community-acquired diseases, such as the staphylococcal infection called MRSA (Methicillin Resistant Staphylococcus Aureus).

Practitioners in the medical field use the stethoscope as much as hands to evaluate patients and administer treatment. Hand sanitizing after examining and treating a patient is primarily aimed at preventing disease transmission, in addition to being a hygienic and healthy habit. Unlike hand sanitizing, there are no current guidelines to sterilize the stethoscope used in between patients.
US 2007/0045039 A I discloses an apparatus for dispensing a cover device, the cover device comprising a frame and a membrane. The apparatus comprises a magazine for storing at least one cover device, the magazine having a mouth through which the at least one cover device may be dispensed.

### Summary of the Invention

The present invention solves the above-mentioned problems by providing a simple and effective way to glove the stethoscope. The disposable stethoscope glove is a new medical device that offers a hygienic way of practicing medicine. The device is designed for single use. It is designed to shield the stethoscope bell or diaphragm, on the anterior surface of the stethoscope, thereby preventing direct contact of the stethoscope to one patient and then to the next. Just like a hand glove, this device serves as a barrier between the stethoscope and the patient, preventing the very stethoscope from becoming a vector of disease and dirt transmission from one patient to another. Due to is cost-effectiveness, it can be utilized by all medial professionals involved in patient care, including but not limited to certified nursing assistants, nurses, physician assistants, and physicians. In this way, it truly and realistically promotes universal precaution in disease prevention and hygienic medical practice. The invention is defined by the appended claims.

The parts of the stethoscope that are used to auscultate the patient may include a wider diaphragm and/or a narrower bell. The pediatric stethoscope diaphragm approximates the size of an adult stethoscope bell. The invention may come in at least two sizes: one size for the adult stethoscope diaphragm, and another one for the adult stethoscope bell and pediatric stethoscope diaphragm. A third size may be smaller to fit a pediatric bell. Additional sizes may be designed to fit alternate stethoscope styles.

The invention may comprise a flexible, elastic, impermeable material that will stretch thinly and fit tightly on the stethoscope's bell or diaphragm. Materials that may be used to produce this device include rubber, latex, nitrile, or flexible vinyl. The material of the glove may also include (*e.g*., by impregnating or coating) an antimicrobial or other appropriate chemical substance. The glove may bear the shape (generally circular) of either the bell or diaphragm but in a smaller dimension and size, and may include an elastic band around its perimeter edge to help maintain shape and fit Once the device is properly applied to the stethoscope, providing a snug fit thereon, only a thin material separates the stethoscope from the patient being examined. This will ensure minimal interference with auscultation. Once the device is used, it is easily removed by pulling the stretchable glove off the diaphragm or bell of the stethoscope.

### Brief Description of the Drawings

The objects, features, and advantages of the present invention will be apparent to one skilled in the art from reading the following description in which:

FIGURE 1 is a perspective view of a medical personnel using an installed stethoscope glove with a patient;
FIGURE 2 is a detail view of a glove unit as it is to be prestretched over a support ring;
FIGURE 3 is a detail view of a wall-mounted box holder having two portions - one for storing the available glove units and one for presenting the mounting tool;
FIGURE 4 is a detail view showing the installation process;
FIGURE 5 is a detail view of a glove installed on a stethoscope bell; and
FIGURE 6 is a detail view illustrating the method for removal of the glove.

### Detailed Description

The following specification describes a stethoscope glove apparatus and method. In the description, specific materials and configurations are set forth in order to provide a more complete understanding of the present invention. But it is understood by those skilled in the art that the present invention can be practiced without those specific details. In some instances, well-known elements are not described precisely so as not to obscure the invention.

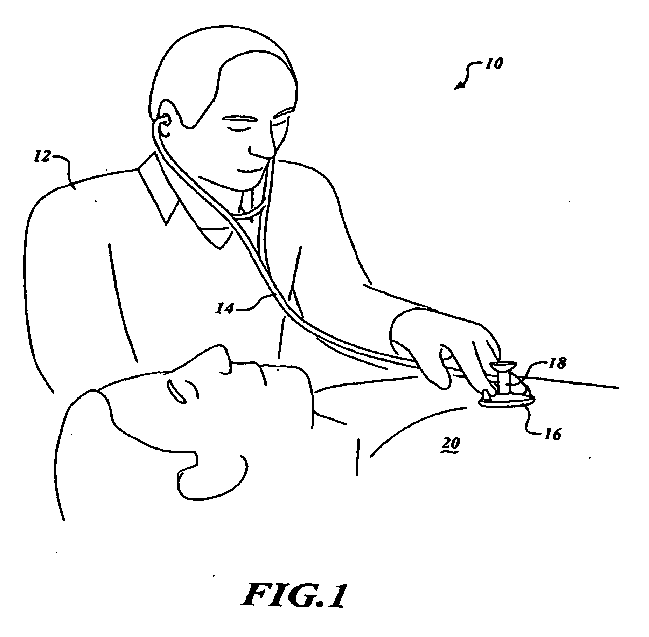

Figure 1 illustrates a proper use of the invention 10 of the stethoscope glove. The figure shows a medical personnel 12 - a physician, nurse, or other care provider-using a stethoscope 14 with the stethoscope glove 16 installed on the bell or diaphragm (stethoscope part) 18. In this case, the medical personnel 12 is using the stethoscope 14 to auscultate the chest of a patient 20, but of course, the glove 16 is also indicated for other appropriate uses of a stethoscope such as in proximity to a patient's back.

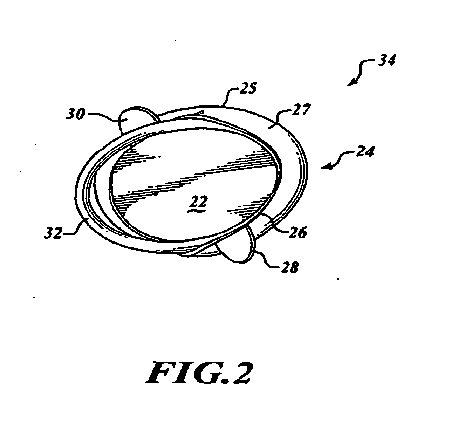

Figure 2 illustrates how the stethoscope glove 16 - which may be made of any appropriate flexible, elastic, impermeable material such as rubber, latex, nitrile, or flexible vinyl that will stretch thinly and fit tightly on the stethoscope's bell or diaphragm - is originally formed in a generally flat, generally circular gasket or sheath with a first surface 22 (which when properly installed will be next to the patient's skin) and a second surface 24 (which will install next to the stethoscope part). An outer rim 25 and a perimeter edge 26 define a lip 27. The perimeter edge 26 may comprise a strip or strips of elastic to enhance its shape memory and is smaller in diameter than the outer rim. There are opposing tabs 28 and 30 extending outwardly from the perimeter edge. A sturdy, generally circular support ring 32 - typically made of plastic - is placed against the first side 22 of the glove, just inside the perimeter edge 26, and the glove 16 is then stretched over the ring 32 to form the prestretched glove unit 34. The diameter of the outer rim 25 should be slightly larger than the diameter of the support ring 32 (which in turn is slightly larger than the diameter of the applicable stethoscope part 18) so that the unit 34 will be formed with the perimeter edge 26 and the opposing tabs 28 and 30 extending outwardly from the perimeter edge and away from the second side 24 of the glove 16. The glove 16 may further define a generally circular ridge or groove on the first surface 22 just inside the perimeter edge (which is preferably slightly smaller in diameter than the support ring) to enhance the fit of the glove 16 around the ring 32. Prestretching the glove in this way may help to ensure that the glove material is uniform in thickness across the stethoscope part to permit high quality auscultation.

Because stethoscopes come in various sizes and styles, including adult and pediatric designs, there may be various sizes of glove units. Furthermore, either the bell or the diaphragm of the stethoscope may be used for auscultation in differing circumstances. For each application, the support ring should be made to have a slightly larger diameter than the diameter of the stethoscope part - bell or diaphragm - intended to be gloved. The generally circular glove is then made to fit the support ring and so will also fit snugly around the stethoscope part upon proper installation.

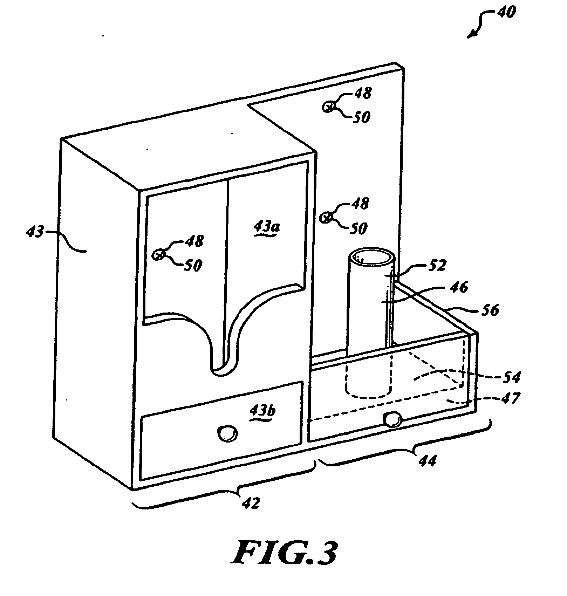

Figure 3 illustrates one example of a box holder 40, which may be wall mounted, that provides easy access to the glove units, a simple mounting tool for applying the glove to the stethoscope part, and a method of waste collection and removal of the spent support rings. The holder 40 need not be in the shape of a box - *e.g.*, it could be oval, round, rectangular, or of some irregular shape - and it also could sit on a horizontal countertop or shelf instead of being wall-mounted, but the options illustrated here have been chosen for explanatory purposes. However, regardless of the shape, the holder 40 should have at least two portions - a first portion 42 for storing a plurality of prestretched glove units 34 and a second portion 44 for presenting the mounting tool 46. The box holder 40 may also comprise means for wall mounting 48, such as screws 50. Wall mounting means could also include adhesive or another fastener such as nails, hook-and-loop strips, etc.

The first portion 42 may have a receptacle 43 that is large enough to hold a reasonable supply of loosely packed glove units 34, and the receptacle 43 should have an opening 43a that is large enough to allow easy access to the units. The opening could be simply a large opening 43a at the top of the receptacle large enough for a hand to easily reach in and select a glove unit, or a door 43b (e.g., hinged or slidable) at the bottom of the receptacle 43 to provide access to the glove units. What is reasonable will, of course, vary depending upon the size and purpose of the facility, the number of box holders and their placement in the building, and the frequency of different patients. The idea is that enough glove units be available for each care provider to use a fresh, clean glove every time he or she auscultates a different patient.

The second portion 44 is to be located in close proximity to the first portion and will present a mounting tool 46. The mounting tool 46 shown is simply a generally vertical (i.e., upright) cylinder (which is to be hollow at least at the upper end 52) and is typically removably fixed to a horizontal shelf 47 of the box holder 40 such that its upper end 52 is easily accessible and available for mounting the glove units. The tool 46 is removable from the box holder so that the support rings 32 that collect around the mounting tool can be discarded periodically. In the illustration of Figure 3, the mounting tool is fixed to a base 54 that can slide out of the shelf 47 so that the rings can be discarded. The short walls 56 are explained further in the following paragraph and may be used to help contain the spent support rings. The box holder can be designed to accommodate various sizes of glove units to fit various sizes of stethoscope parts. In that case, the first portion may be further partitioned and labeled to store more than one size of unit, and the second portion may present more than one differently sized mounting tool.

The box holder may also be supplied as part of a stethoscope glove "installation station" kit along with a plurality of prestretched stethoscope glove units. The kit may further contain mounting hardware for wall mounting the box holder if desired. The box holder of the kit - regardless of its shape or size - should comprise a first portion including a receptacle for the glove units (as described in the foregoing paragraph), and a second portion including a mounting tool (also as described in the foregoing paragraph). The kit may be provided with a plurality of stethoscope glove units already loosely packed in the receptacle of the first portion, so that it is ready for use. The kit may be provided in various styles for different applications, and the generally horizontal shelf of the second portion of the box holder may or may not have the mounting tool removably attached thereto. Instructions for use will be included in the kit.

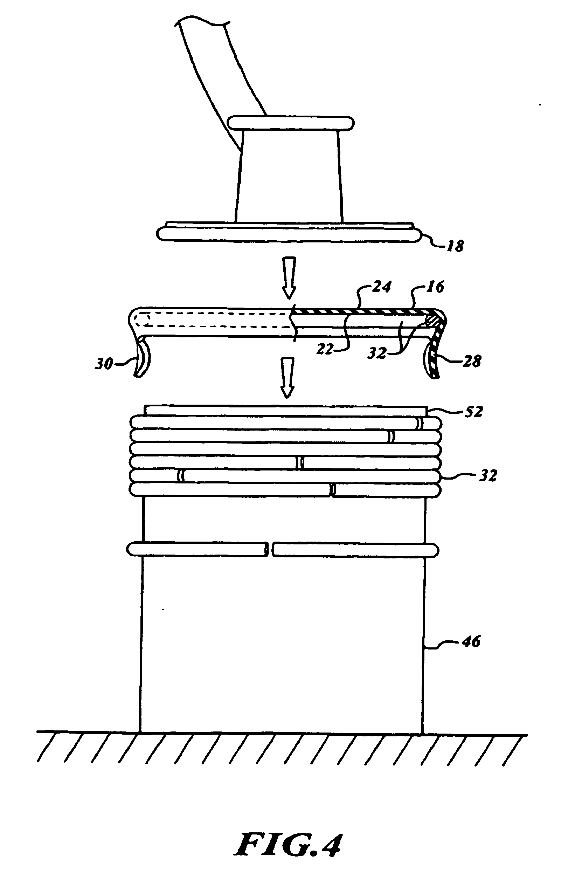

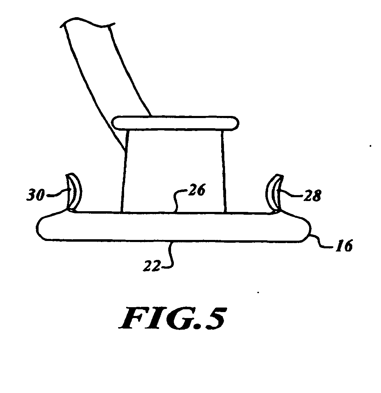

Figure 4 shows how a glove 16 is installed on a stethoscope part 18 - bell or diaphragm in order to prevent disease transmission. The user selects an appropriate stethoscope glove unit 34 from the first portion 42 of the box holder and applies it to the mounting tool 46 by placing it on top of the upper end 52 of the mounting tool 46 with the first surface 22 of the glove 16 facing downward toward the tool. The relative diameters of the mounting tool and the support ring of the stethoscope glove may be such that the support ring (and not the glove itself) will come into contact with the mounting tool. In an appropriately sized mounting tool, the diameter of the upper end 52 will be smaller than the perimeter edge 26 of the glove unit. The user then brings the stethoscope part 18 (whose diameter is slightly smaller than that of the cylinder so that the part will fit inside the hollow upper end) into close proximity of the glove unit - preferably as centered as possible - and gently taps it onto the second surface 24 of the glove proximate the ring 32 of the unit. Upon this tapping, the ring will release the glove 16, which will separate from the ring to wrap around the stethoscope part 18 with the tabs 28 and 30 extending back away from the anterior surface of the stethoscope part (as shown in the next figure 5). The second surface 24 of the glove 16 will now be installed adjacent the stethoscope part, with the first side 22 facing out ready to contact a patient's skin. The support ring (whose diameter is slightly larger than that of the cylinder) will simply fall down around the cylinder of the mounting tool. The rings 32 may be split rings as shown in the illustration, to facilitate their falling down around the cylinder of the mounting tool. When a number of rings have accumulated around the cylinder, the mounting tool can be removed from the box holder so that the spent rings can be discarded into an appropriate refuse receptacle. If the mounting tool 46 is fixed upon a slidable (or otherwise removable) base 54 as in the illustration, the base 54 can be removed to discard the rings and then reinstalled into the shelf 47 ready for use. Alternatively, the material of the split ring 32 could be some type of spring steel (or similar material) which will spring back into a more linear shape after releasing the glove 16. In that case, the box holder 40 of Figure 3 may comprise short walls 56 to contain the more linear "rings" around the base of the mounting tool 46.

Figure 5 shows in detail the stethoscope glove16 installed on the bell or diaphragm 18 of a stethoscope. A properly sized glove will result in the perimeter edge 26 being high enough on the stethoscope part to ensure that the patient's skin is always protected. The first side 22 of the glove is facing outwardly from the stethoscope part (on the anterior surface) and ready to contact the skin of a patient for auscultation, and if desired, an antimicrobial gel or other substance can be applied. The installation method provides a snug fit so that the glove is of uniform thickness across the bell or diaphragm offering a more consistent sound quality for the user. This hands-off installation method also provides that the care provider does not need to use his or her fingers to stretch the glove across the bell or diaphragm - therefore maintaining the integrity of the glove's hygiene.

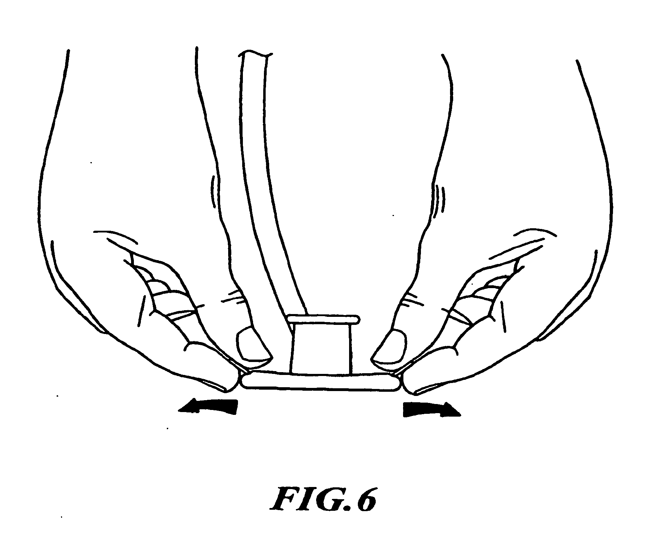

After a single use of the stethoscope glove 16 and in preparation for the next patient, the user may easily remove the glove as illustrated in Figure 6. The user simply grasps the opposing tabs 28 and 30 of the glove, which are sized to be grasped between a human thumb and forefinger, and gently pulls the glove away from the stethoscope. The glove can then be discarded into an appropriate waste receptacle or trash can.

## Claims

1. A stethoscope glove unit (34) comprising:
a generally flat, generally circular stethoscope glove (16) having an outer rim (25), a perimeter edge (26), and a lip (27), prestretched over a generally circular support ring (32),
said stethoscope glove (16) having opposing tabs (28,30) extending outwardly from said perimeter edge (26),
**characterized by** said stethoscope glove unit (34) being configured such that, when said stethoscope glove (16) is installed on a stethoscope part (18) said generally circular support ring (32) is not installed on said stethoscope part (18).

2. The stethoscope glove unit (34) of claim 1 wherein the material of said stethoscope glove (16) is chosen from the group comprising latex, nitrile, rubber, and flexible vinyl.

3. The stethoscope glove unit (34) of claim 1 wherein said outer rim (25) is slightly larger in diameter than said support ring (32).

4. The stethoscope glove unit (34) of claim 1 wherein said stethoscope glove (16) further defines a generally circular groove just inside said perimeter edge (26).

5. The stethoscope glove unit (34) of claim 1 wherein said perimeter edge (26) of said generally circular glove (16) has a slightly smaller diameter than the diameter of said support ring (32).

6. The stethoscope glove unit (34) of claim 1 wherein said support ring (32) is a split ring.

7. The stethoscope glove unit (34) of claim 1 wherein said opposing tabs (28,30) are each of a size to be grasped by a human thumb and forefinger.

8. The stethoscope glove unit (34) of claim 2 wherein said stethoscope glove (16) further includes an antimicrobial substance.

9. A method of installing a stethoscope glove (16) from a stethoscope glove unit (34) on a stethoscope part (18) for the purposes of preventing disease transmission, said stethoscope glove unit (34) comprising a stethoscope glove (16) being prestretched over a support ring (32), said method comprising the steps of:
selecting an appropriate stethoscope glove unit (34) having opposing tabs (28,30);
applying said stethoscope glove unit (34) to a mounting tool (46) attached to a box holder (40);
bringing a stethoscope part (18) to be gloved in close proximity to said stethoscope glove unit (34);
tapping said stethoscope glove unit (34) with said stethoscope part (18) such that said stethoscope glove (16) separates from said support ring (32) and wraps around said stethoscope part (18) with said opposing tabs (28,30) extending therefrom, whereby said support ring (32) is not installed on said stethoscope part (18).

10. The method of claim 9 wherein said stethoscope glove unit (34) is applied to said mounting tool (46) such that said support ring (32) comes into contact with said mounting tool (46).

11. The method of claim 9 further comprising the step of applying an antimicrobial gel to said installed stethoscope glove (16).

12. The method of claim 9 further comprising the step of removing said stethoscope glove (16) from said stethoscope part (18) by grasping said opposing tabs (28,30) and pulling.

13. A kit for a stethoscope glove installation station, comprising:
a box holder (40) comprising a first portion (42) including a receptacle (43) for a plurality of stethoscope glove units (34) and a second portion (44) including a mounting tool (46) for the stethoscope gloves (16) having opposing tabs (28,30); and
a plurality of prestretched stethoscope glove units (34) amounted upon support rings (32). ,
**characterized by** said stethoscope glove unit (34) being configured such that, when said stethoscope glove (16) is installed on a stethoscope part (18), said generally circular support ring (32) is not installed on said stethoscope part (18).

14. The kit of claim 13 wherein said mounting tool (46) is removable from said box holder (40) and comprises a generally vertical cylinder with a hollow upper end (52), the upper end (52) of said cylinder having a slightly smaller diameter than the support ring (32) of said stethoscope glove unit (34).

15. The kit of claim 13 wherein said second portion (44) of said box holder (40) defines a generally horizontal shelf (47) having said mounting tool (46) removably attached thereto.

## Patentansprüche

1. Stethoskophandschuheinheit (34), umfassend:
einen allgemein flachen, allgemein kreisförmigen Stethoskophandschuh (16) mit einem äußeren Rand (25), einer
Umfangskante (26) und einer Lippe (27), vorgedehnt über einem allgemein kreisförmigen Stützring (32),
wobei der Stethoskophandschuh (16) gegenüberliegende Streifen (28, 30) aufweist, welche sich von der Umfangskante (26) nach außen erstrecken,
**dadurch gekennzeichnet, dass** die Stethoskophandschuheinheit (34) derart ausgestaltet ist, dass, wenn der Ste-thoskophandschuh (16) auf einem Stethoskopteil (18) in-stalliert wird, der allgemein kreisförmige Stützring (32) nicht an dem Stethoskopteil (18) installiert wird.

2. Stethoskophandschuheinheit (34) nach Anspruch 1, wobei das Material des Stethoskophandschuhs (16) ausgewählt ist aus der Gruppe, welche Latex, Nitril, Gummi und flexibles Vinyl umfasst.

3. Stethoskophandschuheinheit (34) nach Anspruch 1, wobei der äußere Rand (25) im Durchmesser geringfügig größer ist als der Stützring (32).

4. Stethoskophandschuheinheit (34) nach Anspruch 1, wobei der Stethoskophandschuh (16) darüber hinaus eine allgemein kreisförmige Rille gerade innerhalb der Umfangskante (26) definiert.

5. Stethoskophandschuheinheit (34) nach Anspruch 1, wobei die Umfangskante (26) des allgemein kreisförmigen Handschuhs (16) einen geringfügig kleineren Durchmesser als der Durchmesser des Stützrings (32) aufweist.

6. Stethoskophandschuheinheit (34) nach Anspruch 1, wobei der Stützring (32) ein Spaltring ist.

7. Stethoskophandschuheinheit (34) nach Anspruch 1, wobei die gegenüberliegenden Streifen (28, 30) jeweils eine Größe zum Greifen durch einen menschlichen Daumen und Zeigefinger aufweisen.

8. Stethoskophandschuheinheit (34) nach Anspruch 2, wobei der Stethoskophandschuh (16) darüber hinaus eine antimikrobielle Substanz beinhaltet.

9. Verfahren zur Installation eines Stethoskophandschuhs (16) ausgehend von einer Stethoskophandschuheinheit (34) an einem Stethoskopteil (18), zum Zwecke einer Vermeidung von Krankheitsübertragung, wobei die Stethoskophandschuheinheit (34) einen Stethoskophandschuh (16) umfasst, welcher über einen Stützring (32) vorgedehnt ist, wobei das Verfahren die Schritte umfasst:
Auswählen einer geeigneten Stethoskophandschuheinheit (34) mit gegenüberliegenden Streifen (28, 30);
Applizieren der Stethoskophandschuheinheit (34) an ein Anbringungswerkzeug (46), welches an einer Behälterhalterung (40) angebracht ist;
Bringen eines zu behandschuhenden Stethoskopteils (18) in unmittelbare Nähe zu der Stethoskophandschuheinheit (34);
Antippen der Stethoskophandschuheinheit (34) mit dem Stethoskopteil (18), sodass der Stethoskophandschuh (16) sich von dem Stützring (32) trennt und um das Stethoskopteil (18) wickelt, wobei die gegenüberliegenden Streifen (28, 30) sich davon erstrecken, wobei der Stützring (32) nicht an dem Stethoskopteil (18) installiert wird.

10. Verfahren nach Anspruch 9, wobei die Stethoskophandschuheinheit (34) derart an dem Anbringungswerkzeug (46) angebracht ist, dass der Stützring (32) mit dem Anbringungswerkzeug (46) in Kontakt kommt.

11. Verfahren nach Anspruch 9, darüber hinaus umfassend den Schritt eines Applizierens eines antimikrobiellen Gels auf den installierten Stethoskophandschuh (16).

12. Verfahren nach Anspruch 9, darüber hinaus umfassend den Schritt eines Entfernens des Stethoskophandschuhs (16) von dem Stethoskopteil (18) durch Greifen der gegenüberliegenden Streifen (28, 30) und Ziehen.

13. Kit für eine Stethoskophandschuhinstallationsstation, umfassend:
eine Behälterhalterung (40), umfassend einen ersten Abschnitt (42), welcher einen Behälter (43) für eine Vielzahl von Stethoskophandschuheinheiten (34) beinhaltet, und einen zweiten Abschnitt (44), welcher ein Anbringungswerkzeug (46) für die Stethoskophandschuhe (16) mit gegenüberliegenden Streifen (28, 30) beinhaltet; und
eine Vielzahl von vorgedehnten Stethoskophandschuheinheiten (34), welche auf Stützringen (32) angebracht sind,
**dadurch gekennzeichnet, dass** die Stethoskophandschuheinheit (34) derart ausgestaltet ist, dass, wenn der Stethoskophandschuh (16) an einem Stethoskopteil (18) installiert wird, der allgemein kreisförmige Stützring (32) nicht an dem Stethoskopteil (18) installiert wird.

14. Kit nach Anspruch 13, wobei das Anbringungswerkzeug (46) von der Behälterhalterung (40) entfernbar ist und einen allgemein vertikalen Zylinder mit einem hohlen oberen Ende (52) umfasst, wobei das obere Ende (52) des Zylinders einen geringfügig kleineren Durchmesser als der Stützring (32) der Stethoskophandschuheinheit (34) aufweist.

15. Kit nach Anspruch 13, wobei der zweite Abschnitt (44) der Behälterhalterung (40) ein allgemein horizontales Bord (47) definiert, an welchem das Anbringungswerkzeug (46) entfernbar angebracht ist.

## Revendications

1. Unité de protection de stéthoscope (34) comprenant :
une protection de stéthoscope généralement plate et généralement circulaire (16) ayant un rebord extérieur (25), un bord périphérique (26) et une lèvre (27), pré-étirée sur un anneau de support généralement circulaire (32),
ladite protection de stéthoscope (16) ayant des pattes opposées (28, 30) s'étendant vers l'extérieur depuis ledit bord périphérique (26),
**caractérisée en ce que** ladite unité de protection de stéthoscope (34) est configurée de telle manière que, quand ladite protection de stéthoscope (16) est installée sur une partie de stéthoscope (18), ledit anneau de support généralement circulaire (32) n'est pas installé sur ladite partie de stéthoscope (18).

2. Unité de protection de stéthoscope (34) selon la revendication 1, dans laquelle le matériau de ladite protection de stéthoscope (16) est choisi dans le groupe comprenant le latex, le nitrile, le caoutchouc et le vinyle souple.

3. Unité de protection de stéthoscope (34) selon la revendication 1, dans laquelle ledit rebord extérieur (25) est de diamètre légèrement plus grand que ledit anneau de support (32).

4. Unité de protection de stéthoscope (34) selon la revendication 1, dans laquelle ladite protection de stéthoscope (16) définit en outre une rainure généralement circulaire à l'intérieur dudit bord périphérique (26), à proximité immédiate de celui-ci.

5. Unité de protection de stéthoscope (34) selon la revendication 1, dans laquelle ledit bord périphérique (26) de ladite protection généralement circulaire (16) a un diamètre légèrement inférieur au diamètre dudit anneau de support (32).

6. Unité de protection de stéthoscope (34) selon la revendication 1, dans laquelle ledit anneau de support (32) est un anneau fendu.

7. Unité de protection de stéthoscope (34) selon la revendication 1, dans laquelle lesdites pattes opposées (28, 30) ont chacune une taille qui leur permet d'être saisies par un pouce et un index humains.

8. Unité de protection de stéthoscope (34) selon la revendication 2, dans laquelle ladite protection de stéthoscope (16) comprend en outre une substance antimicrobienne.

9. Procédé d'installation d'une protection de stéthoscope (16) provenant d'une unité de protection de stéthoscope (34) sur une partie de stéthoscope (18) dans le but de prévenir la transmission de maladies, ladite unité de protection de stéthoscope (34) comprenant une protection de stéthoscope (16) qui est pré-étirée sur un anneau de support (32), ledit procédé comprenant les étapes suivantes :
sélectionner une unité de protection de stéthoscope appropriée (34) ayant des pattes opposées (28, 30) ;
appliquer ladite unité de protection de stéthoscope (34) sur un outil de montage (46) monté sur un support de boîte (40) ;
amener une partie de stéthoscope (18) à protéger à proximité étroite de ladite unité de protection de stéthoscope (34) ;
taper ladite unité de protection de stéthoscope (34) avec ladite partie de stéthoscope (18) de telle manière que ladite protection de stéthoscope (16) se sépare dudit anneau de support (32) et s'enroule autour de ladite partie de stéthoscope (18) avec lesdites pattes opposées (28, 30) s'étendant depuis celle-ci, moyennant quoi ledit anneau de support (32) n'est pas installé sur ladite partie de stéthoscope (18).

10. Procédé selon la revendication 9, dans lequel ladite unité de protection de stéthoscope (34) est appliquée sur ledit outil de montage (46) de telle manière que ledit anneau de support (32) vient au contact dudit outil de montage (46).

11. Procédé selon la revendication 9, comprenant en outre l'étape consistant à appliquer un gel antimicrobien sur ladite protection de stéthoscope installée (16).

12. Procédé selon la revendication 9, comprenant en outre l'étape consistant à retirer ladite protection de stéthoscope (16) de ladite partie de stéthoscope (18) en saisissant lesdites pattes opposées (28, 30) et en tirant.

13. Nécessaire pour station d'installation de protection de stéthoscope, comprenant :
un support de boîte (40) comprenant une première partie (42) comportant un boîtier (43) destiné à recevoir une pluralité d'unités de protection de stéthoscope (34) et une deuxième partie (44) comportant un outil de montage (46) pour les protections de stéthoscope (16) ayant des pattes opposées (28, 30) ; et
une pluralité d'unités de protection de stéthoscope pré-étirées (34) montées sur des anneaux de support (32),
**caractérisé en ce que** ladite unité de protection de stéthoscope (34) est configurée de telle manière que, quand ladite protection de stéthoscope (16) est installée sur une partie de stéthoscope (18), ledit anneau de support généralement circulaire (32) n'est pas installé sur ladite partie de stéthoscope (18).

14. Nécessaire selon la revendication 13, dans lequel ledit outil de montage (46) peut être retiré dudit support de boîte (40) et comprend un cylindre généralement vertical ayant une extrémité supérieure creuse (52), l'extrémité supérieure (52) dudit cylindre ayant un diamètre légèrement inférieur à celui de l'anneau de support (32) de ladite unité de protection de stéthoscope (34).

15. Nécessaire selon la revendication 13, dans lequel ladite deuxième partie (44) dudit support de boîte (40) définit une étagère généralement horizontale (47) à laquelle est fixé ledit outil de montage (46) de manière amovible.
